(19) Europäisches Patentamt
European Patent Office
Office européen des brevets

(11) EP 1 932 548 A1

(12) EUROPEAN PATENT APPLICATION
published in accordance with Art. 153(4) EPC

(43) Date of publication:
**18.06.2008 Bulletin 2008/25**

(21) Application number: **06781124.0**

(22) Date of filing: **14.07.2006**

(51) Int Cl.:
***A61L 27/00*** (2006.01) ***C12N 5/06*** (2006.01)

(86) International application number:
**PCT/JP2006/314096**

(87) International publication number:
**WO 2007/039973 (12.04.2007 Gazette 2007/15)**

(84) Designated Contracting States:
**DE FR GB**

(30) Priority: **06.10.2005 JP 2005294058**

(71) Applicants:
- **Olympus Corporation**
  **Tokyo 151-0072 (JP)**
- **Tsuchiya, Toshie**
  **Setagaya-ku**
  **Tokyo**
  **158-0094 (JP)**
- **Tamai, Masato**
  **Yokohama-shi**
  **Kanagawa**
  **227-0044 (JP)**

(72) Inventors:
- **TSUCHIYA, Toshie**
  **Tokyo 158-0094 (JP)**
- **TAMAI, Masato**
  **Yokohama-shi**
  **Kanagawa 227-0044 (JP)**
- **HAKAMATSUKA, Yasuharu**
  **Akishima-shi**
  **Tokyo 196-0033 (JP)**
- **TAMURA, Tomoaki**
  **Hino-shi**
  **Tokyo 191-0032 (JP)**

(74) Representative: **von Hellfeld, Axel et al**
**Wuesthoff & Wuesthoff**
**Patent- und Rechtsanwälte**
**Schweigerstrasse 2**
**81541 München (DE)**

# (54) REINFORCING AGENT FOR BIOLOGICAL TISSUE, METHOD OF PRODUCING THE SAME, UTILIZATION OF THE SAME AND METHOD OF CULTURING CELLS

(57) A method of producing a biological tissue-reinforcing material, which is applicable to a site required to be reinforced and which enables to improve the working properties for transplantation, comprising the step S1 of coprecipitating ammonium hydrogen phosphate with calcium nitrate in the presence of sulfuric acid.

EP 1 932 548 A1

## Description

Technical Field

The present invention relates to a biological tissue-reinforcing material, a method of producing the same, use of the same, and a method of culturing cells.

Background Art

Conventionally, hydroxyapatite (HAP) and β-tricalcium phosphate (βTCP) are known as artificial bone-reinforcing materials, which are a type of biological tissue-reinforcing material (for example, refer to Non-patent Document 1). Recently, it has been reported that calcium sulfate can also be used as a bone-reinforcing material (for example, refer to Non-patent Document 2).
Non-patent Document 1:

> Uemura et. al., "Tissue engineering in bone using biodegradable βTCP porous material - A new material strengthened in vivo: Osferion", Medical Asahi, The Asahi Shimbun Company, October 1, 2001, Vol. 30, No. 10, p.46-49.

Non-patent Document 2:

> Raffy Mirzayan et al, "The use of calcium sulfate in the treatment of benign bone lesions", The Journal of bone and joint surgery, vol. 83-A, No. 3, March 2001, p.355-358.

Disclosure of Invention

However, calcium sulfate is a powder and can not be formed into a sintered compact as calcium phosphate can be, thus causing an inconvenience of being inapplicable to a site required to be reinforced. Moreover, since calcium sulfate is a powder, there is a problem in that the working properties are unsatisfactory for transplantation.
The present invention takes the above problems into consideration, with an object of providing a biological tissue-reinforcing material which is applicable to a site required to be reinforced and which enables to improve the working properties for transplantation, a method of producing the same, use of the same, and a method of culturing cells.
The present invention employs the following solutions in order to achieve the above object.
The present invention provides a biological tissue-reinforcing material comprising sulfate group-containing calcium phosphate.
In the invention, the preparation is preferably performed so that the ratio of sulfur (S) to phosphorus (P) satisfies $1/2 \leq P/(S+P) \leq 5/6$.
The biological reinforcing material may additionally contain a vivo-derived substance.
Moreover, the present invention provides a method of producing a biological tissue-reinforcing material, comprising the step of coprecipitating ammonium hydrogen phosphate with calcium nitrate in the presence of sulfuric acid.
Further, the present invention provides a method of culturing cells, comprising the step of combining the biological tissue-reinforcing material with a vivo-derived substance.
Moreover, the present invention provides use of the biological tissue-reinforcing material as a cell culture substrate in combination with a vivo-derived substance.
In the present invention, the vivo-derived substance may include at least either one of a growth factor and a cytokine.
Moreover, in the present invention, the vivo-derived substance may include a tissue-derived cell.
Furthermore, in the present invention, the vivo-derived substance may include a cell group containing stem cells.
The biological tissue-reinforcing material and the method of culturing cells of the present invention demonstrate effects of being applicable to a site required to be reinforced while enabling to improve the working properties for transplantation. Moreover, according to the production method of the present invention, effects in which phosphorus and sulfur can be homogenously mixed and readily prepared at a determined mixture ratio, are demonstrated.

Brief Description of Drawings

> FIG. 1 is a flowchart showing a method of producing a biological tissue-reinforcing material according to an embodiment of the present invention.
> FIG. 2 shows X-ray analysis results of the biological tissue-reinforcing material according to the present embodiment produced by the production method of FIG. 1.
> FIG. 3 is a graph showing the proliferation potency of osteoblasts seeded and cultured on a pellet of the biological tissue-reinforcing material according to the present embodiment produced by the production method of FIG. 1.
> FIG. 4 is a graph showing the differentiation potency of osteoblasts seeded and cultured on a pellet of the biological tissue-reinforcing material according to the present embodiment produced by the production method of FIG. 1.
> FIG. 5 is a graph showing the proliferation potency of osteoblasts cultured using a medium containing the biological tissue-reinforcing material according to the present embodiment produced by the production method of FIG. 1.
> FIG. 6 is a graph showing the differentiation potency of osteoblasts cultured using a medium containing the biological tissue-reinforcing material according to the present embodiment produced by the production method of FIG. 1.

FIG. 7 is a graph showing results of the proliferation potency assessed by DNA amount when hMSCs were cultured in an extract in Experimental Example 3-1.

FIG. 8 is a graph showing results of the proliferation potency assessed by DNA amount when hMSCs were cultured on a pellet in Experimental Example 3-1.

FIG. 9 is a graph showing results of the differentiation potency assessed by the production amount of alkaline phosphatase when hMSCs were cultured in an extract in Experimental Example 3-2.

FIG. 10 is a graph showing results of the differentiation potency assessed by the production amount of alkaline phosphatase when hMSCs were cultured on a pellet in Experimental Example 3-2.

FIG. 11 is a graph showing results of the differentiation potency assessed by the production amount of osteocalcin when hMSCs were cultured the extract in Experimental Example 3-2.

FIG. 12 is a graph showing results of the differentiation potency assessed by the production amount of osteocalcin when hMSCs were cultured on the pellet in Experimental Example 3-2.

FIG. 13 is a graph showing results of the results of the degree of calcification assessed by the deposition amount of calcium when hMSCs were cultured in an extract in Experimental Example 4.

FIG. 14 is a graph showing the assessment results of the absorption amount of bFGF in Experimental Example 5.

Best Mode for Carrying Out the Invention

Hereunder is a description of a biological tissue-reinforcing material and a method of producing the same according to an embodiment according to the present invention, with reference to FIG. 1 to FIG. 6.

The biological tissue-reinforcing material according to the present embodiment comprises sulfate group-containing calcium phosphate.

As shown in FIG. 1, this biological tissue-reinforcing material can be produced using the following production method.

First, 0.5M sulfuric acid $H_2SO_4$, 0.5M ammonium hydrogen phosphate $(NH_4)_2HPO_4$ aqueous solution, and 0.5M calcium nitrate $Ca(NO_3)_2$ aqueous solution are prepared. Next, thus prepared 0.5M sulfuric acid and 0.5M ammonium hydrogen phosphate aqueous solution are mixed. Then, the obtained mixture is adjusted to pH 10 using 1.0N sodium hydroxide.

Thus prepared mixture is added with 0.5M calcium nitrate aqueous solution gradually dropwise (step S1). At this time, over monitoring the pH of the mixture, 1.0N sodium hydroxide is added to adjust the pH to 10. After the dropwise addition, the mixture is stirred over day and night (24 hours) (step S2). The precipitate is separated by centrifugation and washed with distilled water (step S3). The washed precipitate is filtered (step S4), dried (step S5), and retained in an atmospheric air at 800°C for 2 hours to thereby effect sintering (step S6). By so ding, the biological tissue-reinforcing material of the present embodiment is produced.

In this manner, according to the method of producing a biological tissue-reinforcing material of the present embodiment, since the step S1 of coprecipitating ammonium hydrogen phosphate with calcium nitrate in the presence of sulfuric acid, is included, then a biological tissue-reinforcing material which homogenously contains phosphorus and sulfur at a determined mixture ratio can be readily produced. Moreover, according to thus produced biological tissue-reinforcing material of the present embodiment, the action of the homogenously contained sulfate group brings superior proliferation potency and differentiation potency of osteoblasts to those of conventional HAP, while enabling to retain characteristics of conventional HAP.

In particular, the ratio of sulfur S to phosphorus P preferably satisfies $1/2 \leq P/(S+P) \leq 5/6$. By so doing, equivalent physical properties to those of conventional HAP can be provided.

Moreover, according to the biological tissue-reinforcing material and the method of producing the same of the present embodiment, advantages are provided in which the production is carried out without a need for special raw materials, at an approximately comparative cost to the production cost using conventional HAP.

The biological tissue-reinforcing material according to the present embodiment is surely applicable as a conventional bone-reinforcing material, as well as being applicable to the following directions because of its high osteoblast-activating action.

1. Culture substrate for osteoblasts

Osteoblasts can be cultured at a highly activated state.

2. Substrate for cultured bone

A cultured bone having a high osteoblast activity or a cultured bone rich in the extracellular matrix can be provided.

3. Bone-reinforcing material

A bone-reinforcing material which activates osteoblasts can be provided.

4. Therapeutic agent for osteoporosis

Therapeutic effects can be expected due to the activation of osteoblasts and the recalcification effect of the extracellular matrix.

5. Therapeutic agent for periodontal bone regeneration

Therapeutic effects can be expected due to the activation of osteoblasts and the recalcification effect of the extracellular matrix.

(Example)

Next is a description of Example of the biological tissue-reinforcing material and the method of producing the same according to the above embodiment.
In the present Example, a biological tissue-reinforcing material comprising a sulfuric acid-HAP was produced in accordance with the flowchart shown in Fig. 1.
0.5M sulfuric acid and 0.5M ammonium hydrogen phosphate aqueous solution were mixed so that the ratio of sulfate ions $SO_4^{2-}$ to phosphate ions $PO_4^{3-}$, that is, $X = PO_4^{3-}/(SO_4^{2-}+PO_4^{3-}) = P/\ (S+P)$, satisfies X = 0, 1/6, 2/6, 3/6, 4/6, 5/6, or 6/6, and the obtained mixture was adjusted to pH 10 using 1.0N sodium hydroxide.
FIG. 2 shows X-ray analysis results of thus obtained biological tissue-reinforcing material of the present embodiment having ratios of sulfur to phosphorus X = 1/6 to 5/6, conventional HAP having X = 6/6, and calcium sulfate having X = 0. According to this FIG. 2, it was confirmed that, in cases of X = 3/6 to 5/6, the biological tissue-reinforcing material according to the present embodiment takes an approximately same structure as that of conventional HAP (X = 6/6).
Next, a pellet comprising the biological tissue-reinforcing material according to the present embodiment was produced. The pellet was produced by the following manner. A powder of sulfuric acid-HAP obtained in the drying step S5 of FIG. 1 was put into, for example, a stainless mold, and was press-formed at 30MPa. The formed product was sintered in an atmospheric air at 800°C for 2 hours in the sintering step S6.

(Experimental Example 1)

On thus produced pellet of the biological tissue-reinforcing material according to the present embodiment, normal human osteoblasts were seeded at $4 \times 10^4$ cells/well/mL, and were cultured for a week. The proliferation potency of the resultant osteoblasts is shown in FIG. 3, and the differentiation potency thereof is shown in FIG. 4, respectively in ratios with respect to the control. The control respectively means cells which were directly cultured in a culture dish without seeding.
The proliferation potency was measured with Tetra Color One (450 nm), and the differentiation potency was measured using alkaline phosphatase (ALP) activity through the color reaction of PNP (405nm).
According to this, the pellet of the biological tissue-reinforcing material according to the present embodiment was confirmed to have significantly higher proliferation potency and differentiation potency of osteoblasts as compared to those of conventional HAP (X = 6/6) and calcium sulfate (X = 0/6).

(Experimental Example 2)

Next, the sulfuric acid-HAP powder that had been heat-treated in the sintering step S6 was put in a medium (100 mg/mL), and agitated with a shaker for 72 hours (150 rpm). Then, the obtained liquid was placed in a centrifugal separator and subjected to centrifugation at 3000 rpm for 10 minutes. Thus obtained supernatant was added with osteoblasts ($4 \times 10^4$ cells/well/mL). The proliferation potency of the resultant cultured osteoblasts is shown in FIG. 5, and the differentiation potency thereof is shown in FIG. 6, respectively in ratios with respect to the control. The control respectively means cells which were directly cultured in a culture dish without seeding.
According to this, it was found that even the mere addition of the biological tissue-reinforcing material according to the present embodiment in the medium of osteoblasts was able to increase the proliferation potency and the differentiation potency.

(Experimental Example 3)

A pellet (12 mmφ × 1 mm) comprising the biological tissue-reinforcing material having the ratio of sulfur to phosphorus X = 4/6 (referred to as "$SO_4$-HAp" in the following description and drawings) was produced in the same production method as that of the above embodiment and was used as a sample. Moreover, a pellet (12 mmφ × 1 mm) comprising conventional hydroxyapatite (X = 6/6; abbreviated as "HAp" in the following description and drawings) was used as a comparative sample.
In order to examine the use of $SO_4$-HAp as a scaffold material (culture substrate) in regenerative medicines, using the above samples as scaffold materials (culture substrates), human mesenchymal stem cells (hereunder, abbreviated as "hMSC") were seeded in a bone differentiation-inducing medium containing differentiation inducing factors (dexamethasone, ascorbic acid, and β-glycerophosphoric acid) in a 24-well plate at $2 \times 10^4$ cells/ml/well, and cultured (7 days, 14 days, and 21 days) to examine the proliferation and the differentiation of hMSCs.
Moreover, in order to examine the influence of runoff components of $SO_4$-HAp, extraction treatment (100 mg/ml, 37°C, 3 days) was performed and hMSCs were cultured in the obtained extract (7 days, 14 days, and 21 days).

Experimental Example 3-1: Assessment of hMSC proliferation potency

The hMSC proliferation was quantified through the production of a cell lysate and following measurement of DNA amount in the lysate.
The cell lysate was produced as follows. First, on completion of culture, the medium was discarded and cells were washed with a phosphate buffered saline solution (PBS) twice. Then, trypsin was added thereto (200 μl/well, 37°C, 1 minute) to peel off these cells. PBS was added at 800 μl per each well, and the cell solution was collected and centrifuged (4°C, 1000 rpm, 2 minutes). The supernatant was discarded and the cells were collected. The collected cells were added with 600 μl of a solution containing 2% nonionic surfactant Nonidet (registered trademark) P-40. The solution was cooled in ice and treated with ultrasonic waves for 10 seconds to disrupt these cells.

In the DNA quantification, PicoGreen (registered trademark) ds DNA quantification kit (excitation wavelength (Ex): 485 nm; detection wavelength (Em): 530 nm) manufactured by Molecular Probes was used. FIG. 7 shows the proliferation potency resulting from the culture in the extract, and FIG. 8 shows the proliferation potency resulting from the culture on the pellet, respectively using $SO_4$-HAp and HAp.

Experimental Example 3-2: Assessment of hMSC differentiation potency

The differentiation potency was assessed by the production amounts of alkaline phosphatase and osteocalcin.
The production amount of alkaline phosphatase was measured as follows. On completion of culture, the medium in each well was discarded and cells were washed PBS. Then, 0.1M glycine-NaCl-NaOH buffer solution containing 4 mM p-nitrophosphoric acid, 10 mM $MgCl_2$, and 0.1 mM $ZnCl_2$ was added at 500 μl per each well. After leaving at a room temperature for 5 minutes, the absorbance of the buffer solution (optical density O.D. at the wavelength of 405 nm) was measured.

The production amount of osteocalcin was measured by placing 100 μl of the cell lysate in an antibody plate, and following measurement of the osteocalcin concentration with use of a commercially available ELISA kit (Gla-OC EIA Kit (manufactured by TAKARA BIO INC.)).

FIG. 9 shows the production amount of alkaline phosphatase resulting from the culture in the extract, FIG. 10 shows the production amount of alkaline phosphatase resulting from the culture on the pellet, FIG. 11 shows the production amount of osteocalcin resulting from the culture in the extract, and FIG. 12 shows the production amount of osteocalcin resulting from the culture on the pellet, respectively using $SO_4$-HAp and HAp.

Since hMSCs are said to be immature cells as compared to mature cells such as osteoblasts and are superior in the proliferation potency to mature cells, they attract a lot of attention as a cell source to be used for regenerative medicines. The results of Experimental Example 3 suggest that the combination of the biological tissue-reinforcing material of the present invention with hMSCs or stem cells would optimize the combined cell potency, and the effect thereof is expected to improve the therapeutic effect.

(Experimental Example 4)

On completion of culture in the extract of Experimental Example 3, cells were collected, and then 0.1N hydrochloric acid was added at 500 μl per each well to solve deposited calcium. Subsequently, the calcium concentration of this solution was quantified using Calcium C-Test Wako (Wako Chemicals), to be used as the measured value of the degree of calcification. The measurement results are shown in FIG. 13.

(Experimental Example 5)

A pellet comprising the same $SO_4$-HAp as that of Experimental Example 3 (ratio of sulfur to phosphorus X = 4/6) and a pellet comprising HAp (ratio of sulfur to phosphorus X = 6/6) were respectively put in a serum-free medium containing 500 pg/ml of a basic fibroblast growth factor (hereunder, referred to as "bFGF"; manufactured by Pepro Tech EC Ltd. (London, UK)). These solutions were incubated at 37°C for 7 days, and thereby the absorption of bFGF was examined. The bFGF amount absorbed onto the pellet was measured using a reagent included in a commercially available ELISA kit (Quantikine (registered trademark), Human FGF Basic Immunoassay, R&D Systems Inc., Minneapolis, MN, USA).

First, the incubated pellet was washed with a cleaning solution included in the ELISA kit, and added with 800 μl of bFGF conjugate. After 2 hours, a substrate solution and a stop solution were added in accordance with the protocol of the ELISA kit. The colored solution was transferred in a new well, and the absorbance of the solution at 450 nm was measured. At this time, the bFGF amount absorbed on the well containing no pellet was used as a control value. The measurement results are shown in FIG. 14.

The measurement results showed that $SO_4$-HAp had a higher bFGF adsorption capacity than that of HAp. Growth factors or cytokines have stimulating and activating effects on related cells, and are said to be associated with bone remodeling and actions of inducing stem cells or immunocytes to necessary sites, for example. The results of the present Experimental Examples suggest that the combination of $SO_4$-HAp with a growth factor or the like would provide a long-time sustaining effect on the efficacy of the combined growth factor (sustained-release effect of the growth factor over a long time), and the effect thereof is expected to improve the therapeutic effect.

## Claims

**1.** A biological tissue-reinforcing material, comprising sulfate group-containing calcium phosphate.

**2.** A biological tissue-reinforcing material according to claim 1, wherein the preparation is performed so that a ratio of sulfur (S) to phosphorus (P) satisfies

$$1/2 \leq P/(S+P) \leq 5/6.$$

**3.** A method of producing a biological tissue-reinforcing material, comprising the step of coprecipitating ammonium hydrogen phosphate with calcium nitrate in the presence of sulfuric acid.

**4.** A biological tissue-reinforcing material, having a vivo-derived substance and a substrate which comprises sulfate group-containing calcium phosphate.

**5.** A biological tissue-reinforcing material according to claim 4, wherein the preparation is performed so that a ratio of sulfur (S) to phosphorus (P) satisfies

$$1/2 \leq P/(S+P) \leq 5/6.$$

**6.** A biological tissue-reinforcing material according to either one of claim 4 and claim 5, wherein the vivo-derived substance includes at least either one of a growth factor and a cytokine.

**7.** A biological tissue-reinforcing material according to either one of claim 4 and claim 5, wherein the vivo-derived substance includes a tissue-derived cell.

**8.** A biological tissue-reinforcing material according to either one of claim 4 and claim 5, wherein the vivo-derived substance includes a cell group containing stem cells.

**9.** A method of culturing cells, comprising the step of combining the biological tissue-reinforcing material according to either one of claim 1 and claim 2 with a vivo-derived substance.

**10.** A method of culturing cells according to claim 9, wherein the vivo-derived substance includes at least either one of a growth factor and a cytokine.

**11.** A method of culturing cells according to claim 9, wherein the vivo-derived substance includes a tissue-derived cell.

**12.** A method of culturing cells according to claim 9, wherein the vivo-derived substance includes a cell group containing stem cells.

**13.** Use of the biological tissue-reinforcing material according to either one of claim 1 and claim 2 as a cell culture substrate in combination with a vivo-derived substance.

**14.** The use of a biological tissue-reinforcing material according to claim 13, wherein the vivo-derived substance includes at least either one of a growth factor and a cytokine.

**15.** The use of a biological tissue-reinforcing material according to claim 13, wherein the vivo-derived substance includes a tissue-derived cell.

**16.** The use of a biological tissue-reinforcing material according to claim 13, wherein the vivo-derived substance includes a cell group containing stem cells.

FIG. 1

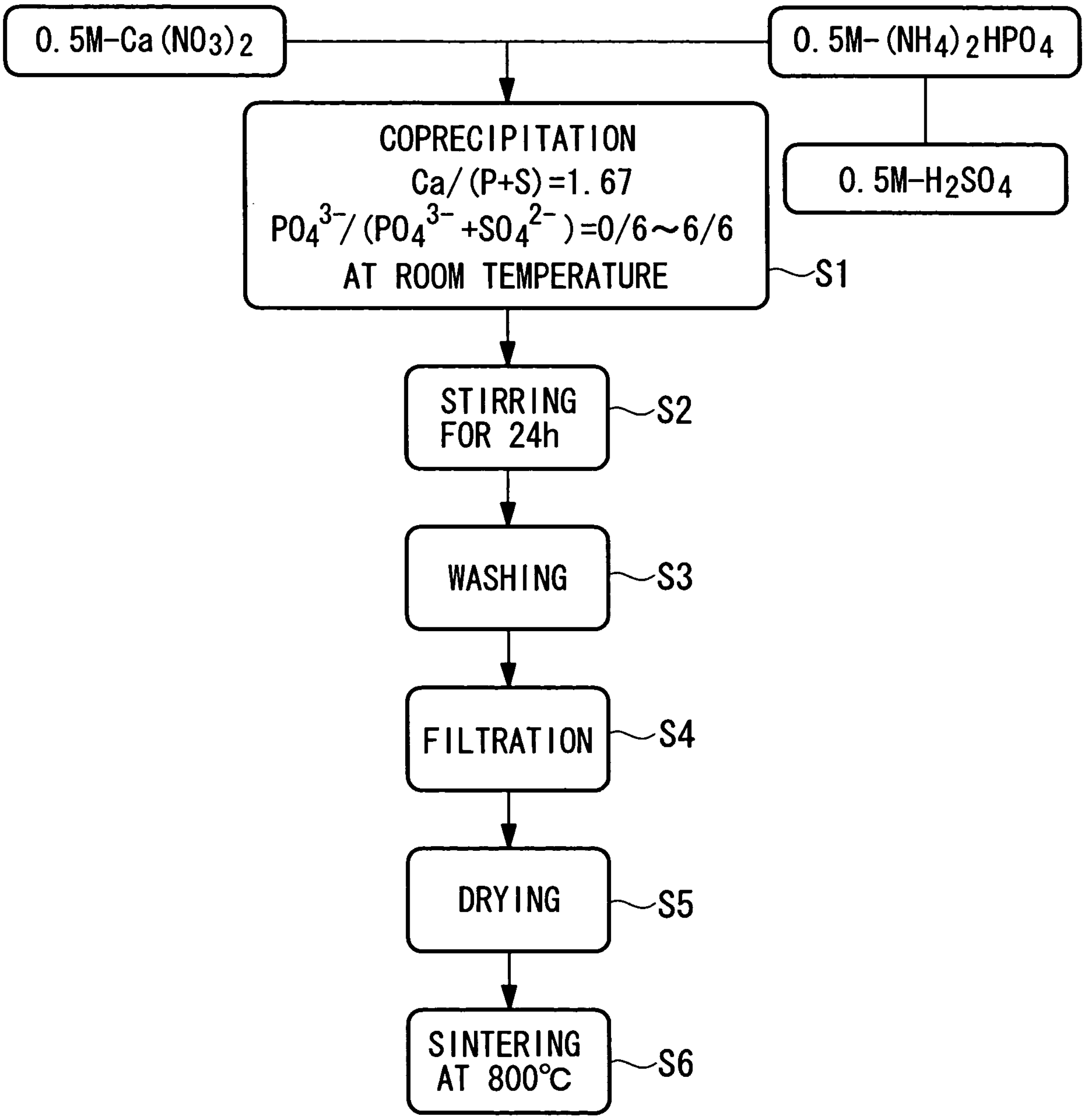
0.5M-Ca(NO3)2
0.5M-(NH4)2HPO4
0.5M-H2SO4
COPRECIPITATION
Ca/(P+S)=1.67
PO4 3-/(PO4 3- +SO4 2-)=0/6~6/6
AT ROOM TEMPERATURE
S1
STIRRING FOR 24h
S2
WASHING
S3
FILTRATION
S4
DRYING
S5
SINTERING AT 800°C
S6

FIG. 2

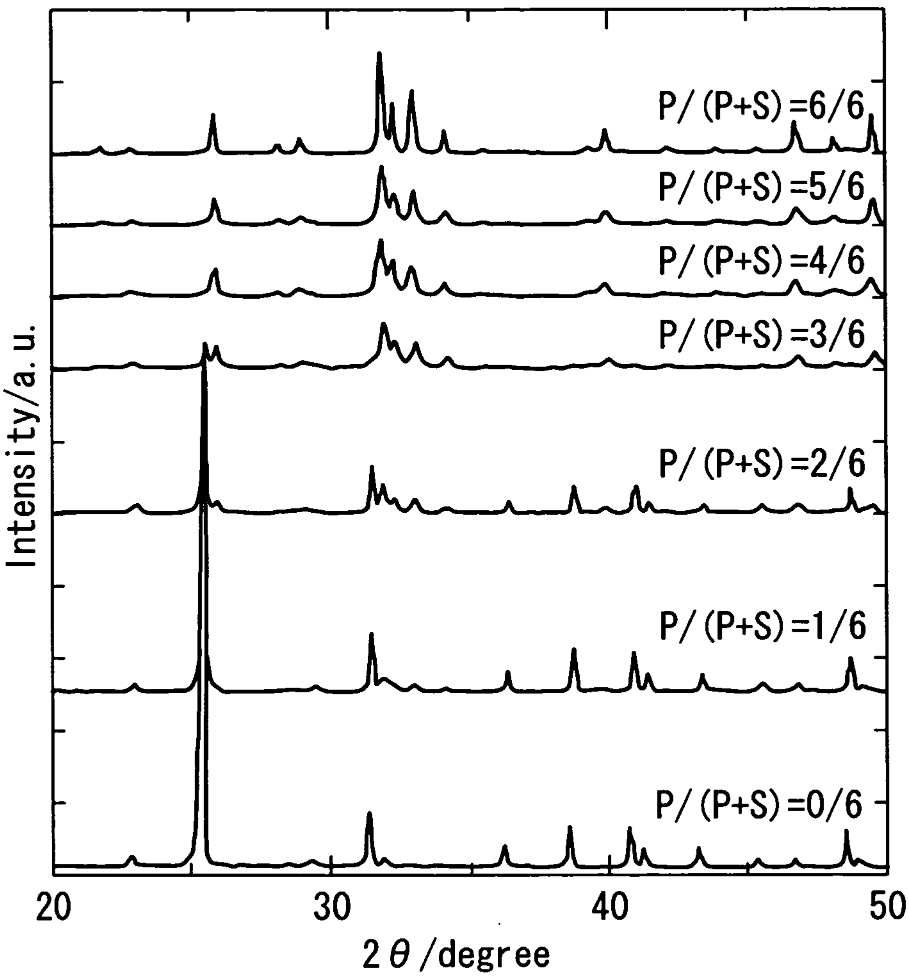
P/(P+S)=6/6
P/(P+S)=5/6
P/(P+S)=4/6
P/(P+S)=3/6
P/(P+S)=2/6
P/(P+S)=1/6
P/(P+S)=0/6
Intensity/a.u.
20
30
40
50
2θ/degree

FIG. 3

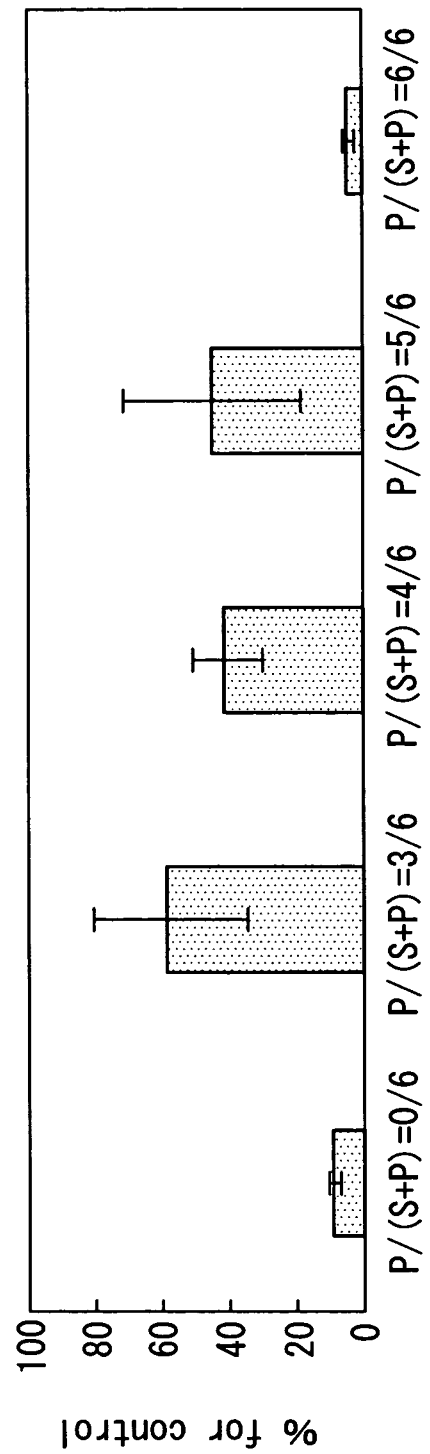

FIG. 4

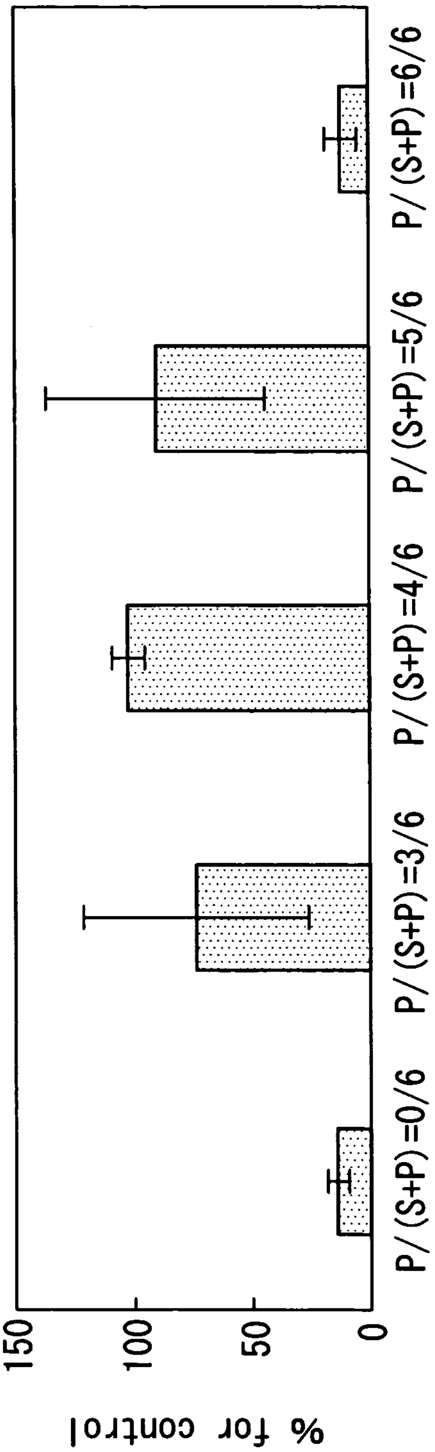

FIG. 5

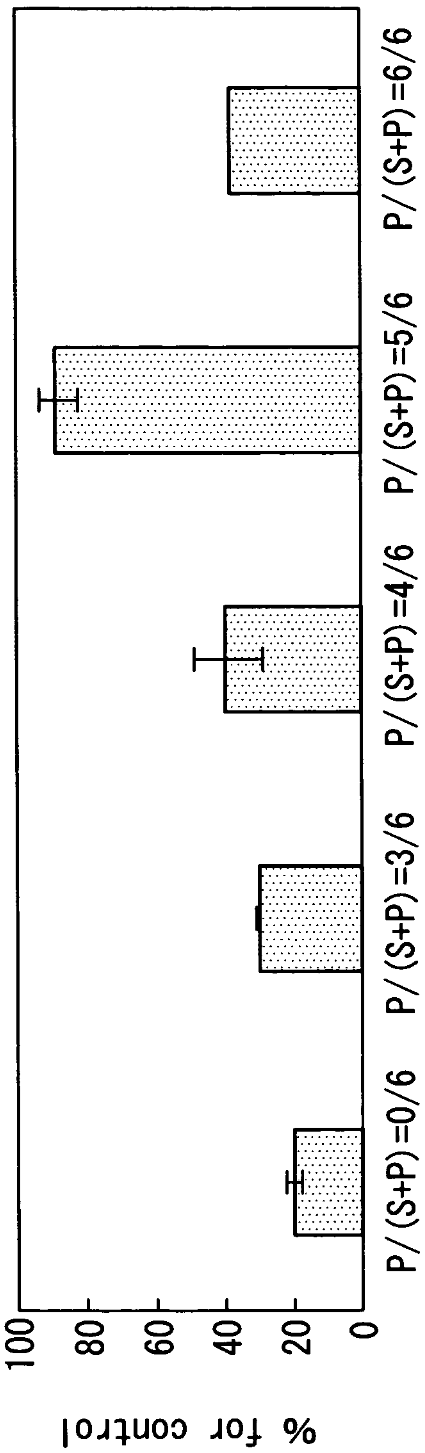

FIG. 6

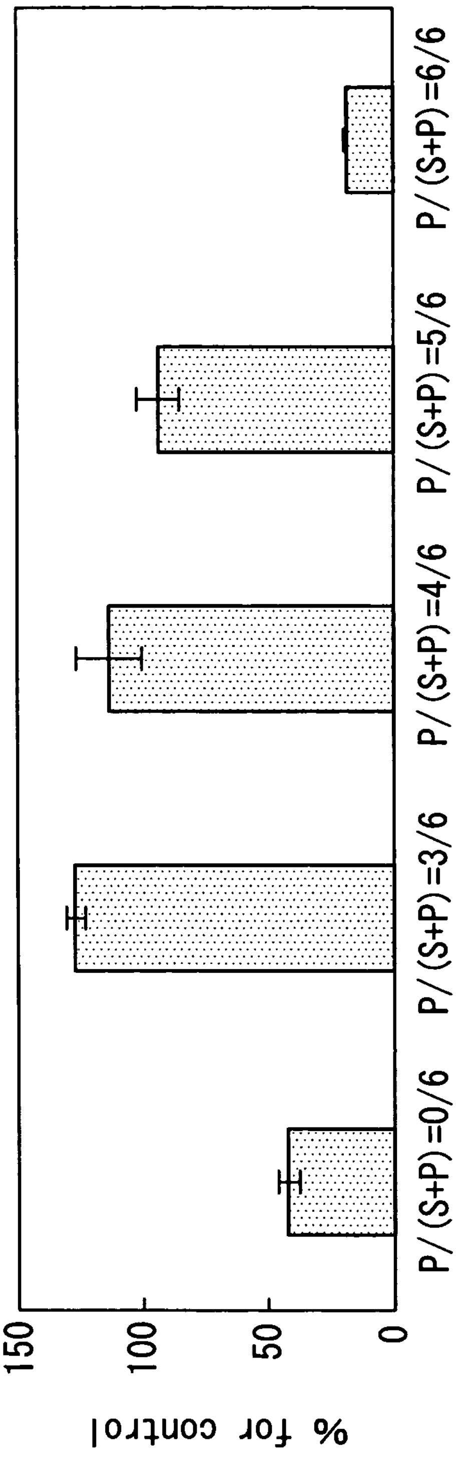

FIG. 7

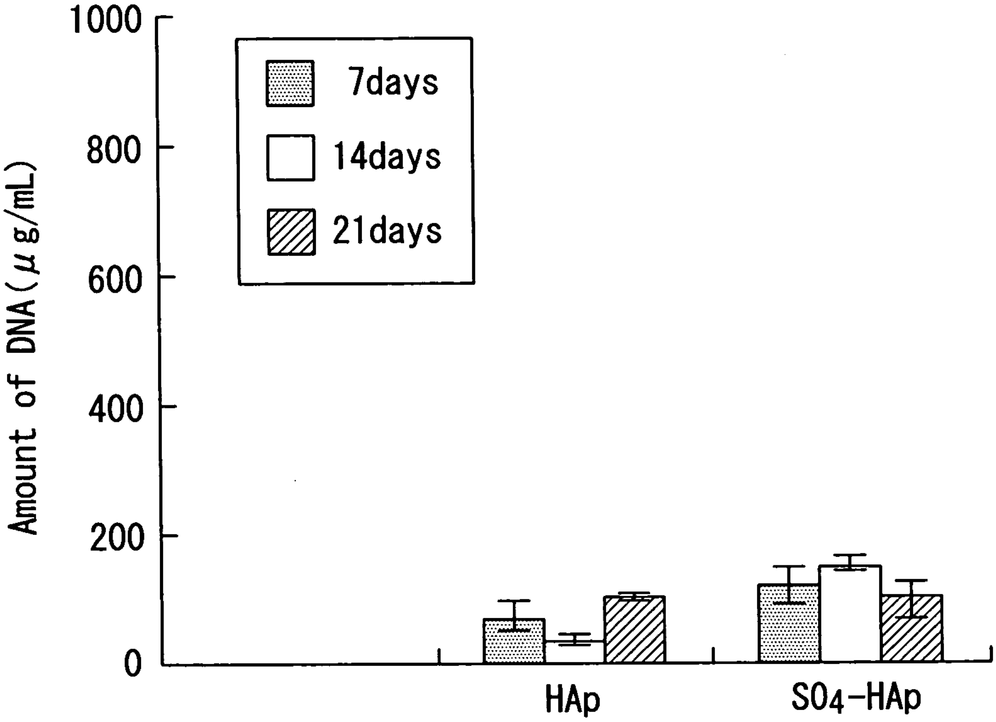
1000
800
600
400
200
0
Amount of DNA (μg/mL)
7days
14days
21days
HAp
SO4-HAp

FIG. 8

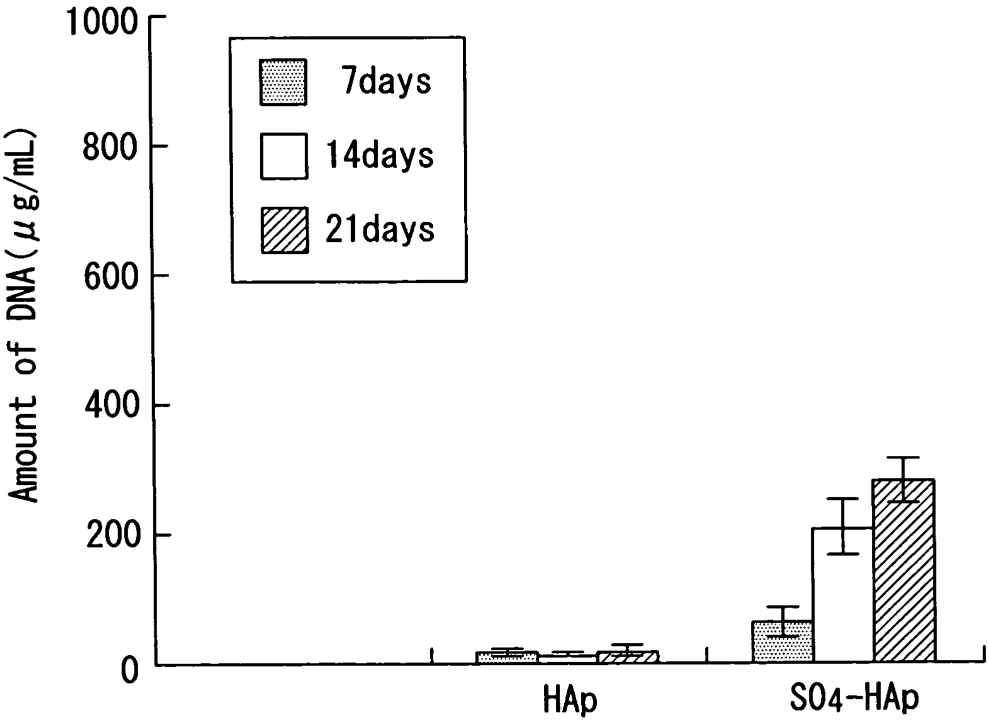
1000
800
600
400
200
0
Amount of DNA (μg/mL)
7days
14days
21days
HAp
SO4-HAp

FIG. 9

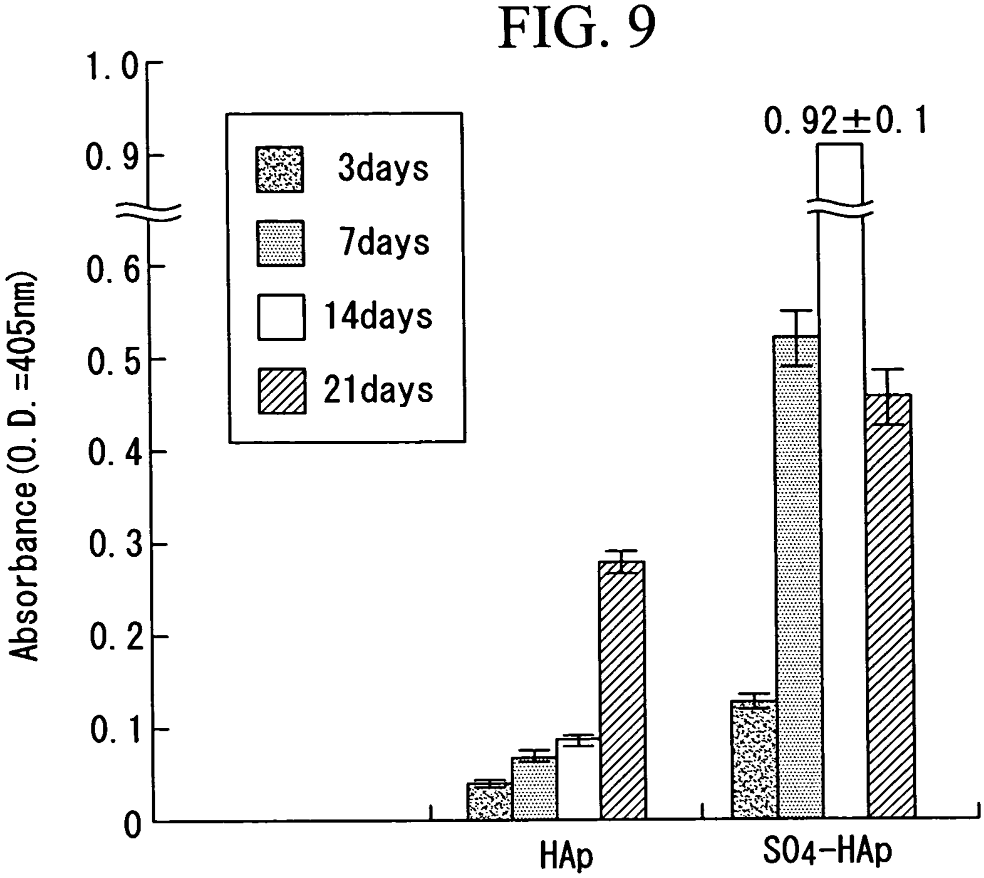
1.0
0.9
0.6
0.5
0.4
0.3
0.2
0.1
0
Absorbance (O. D. =405nm)
3days
7days
14days
21days
0.92±0.1
HAp
SO4-HAp

FIG. 10

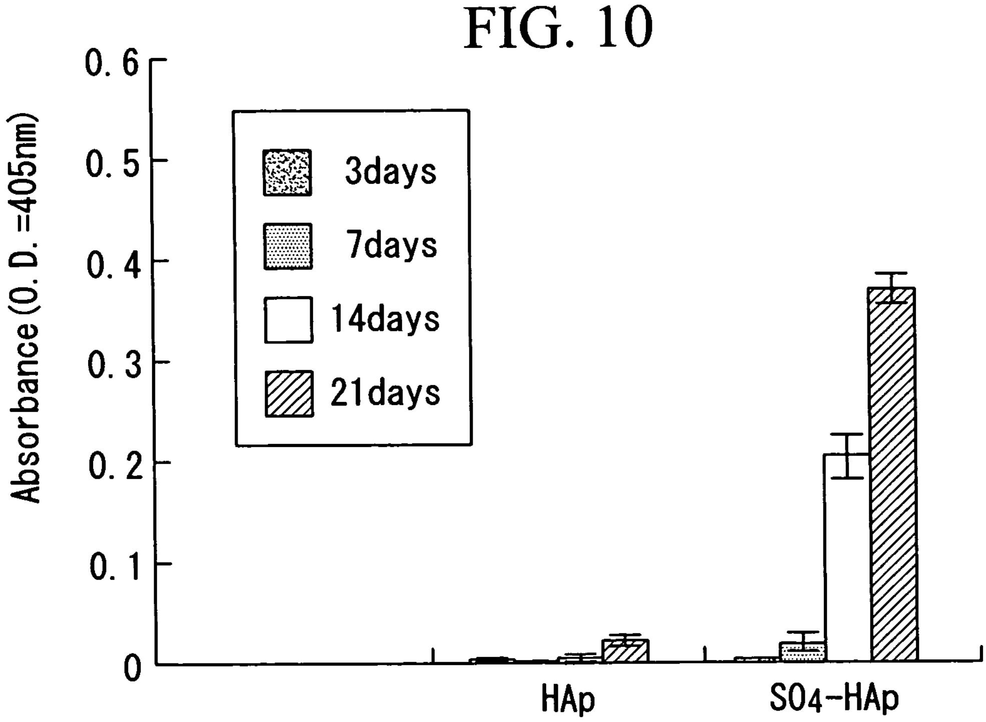
0.6
0.5
0.4
0.3
0.2
0.1
0
Absorbance (O. D. =405nm)
3days
7days
14days
21days
HAp
SO4-HAp

FIG. 11

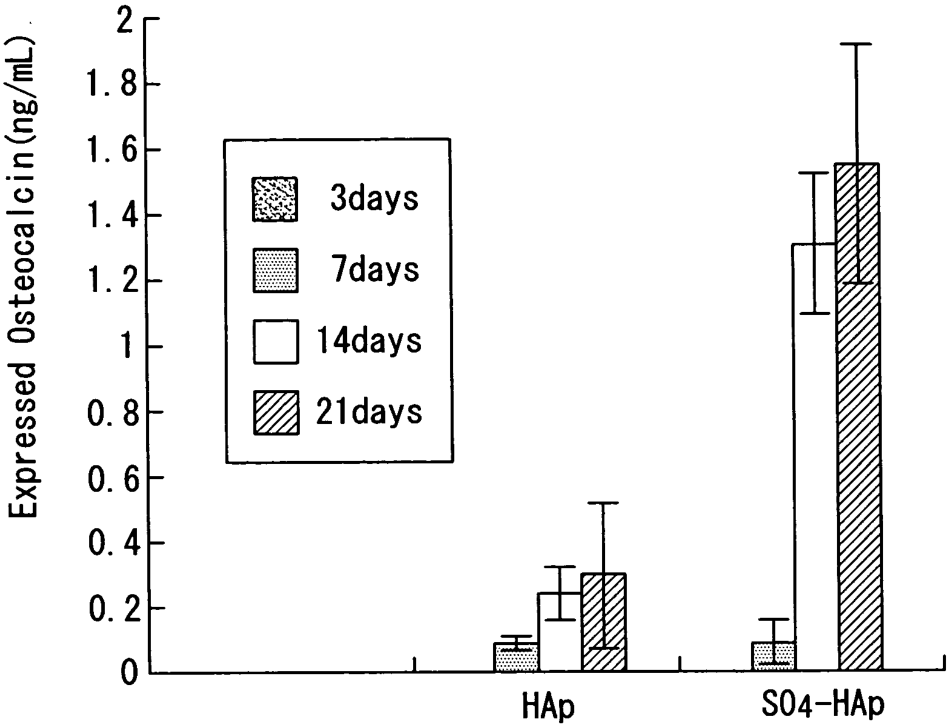
Expressed Osteocalcin(ng/mL)
2
1.8
1.6
1.4
1.2
1
0.8
0.6
0.4
0.2
0
3days
7days
14days
21days
HAp
SO4-HAp

FIG. 12

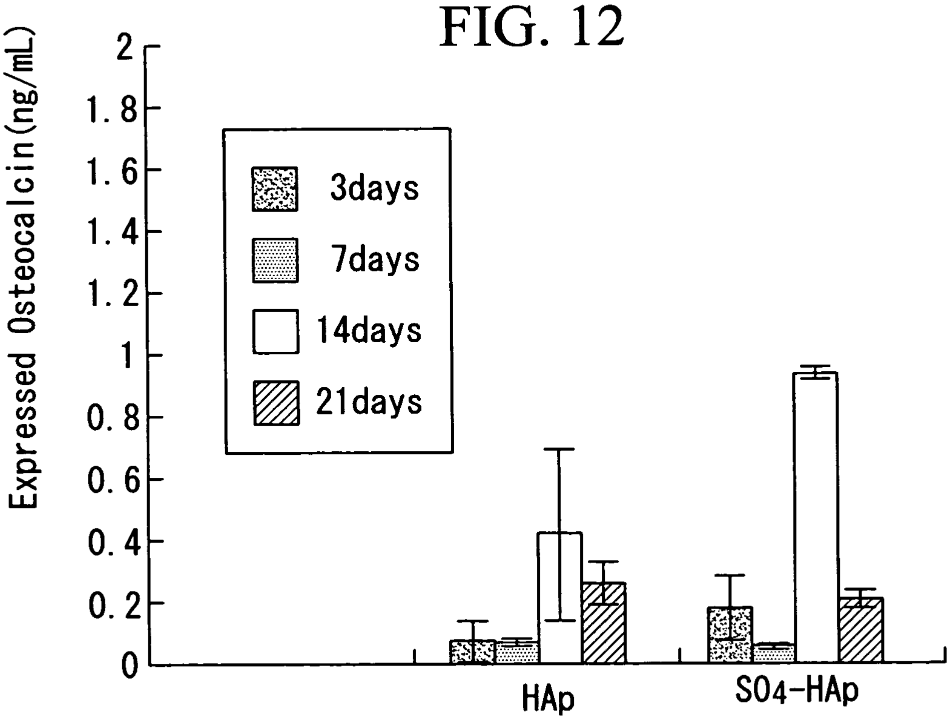
Expressed Osteocalcin(ng/mL)
2
1.8
1.6
1.4
1.2
1
0.8
0.6
0.4
0.2
0
3days
7days
14days
21days
HAp
SO4-HAp

FIG. 13

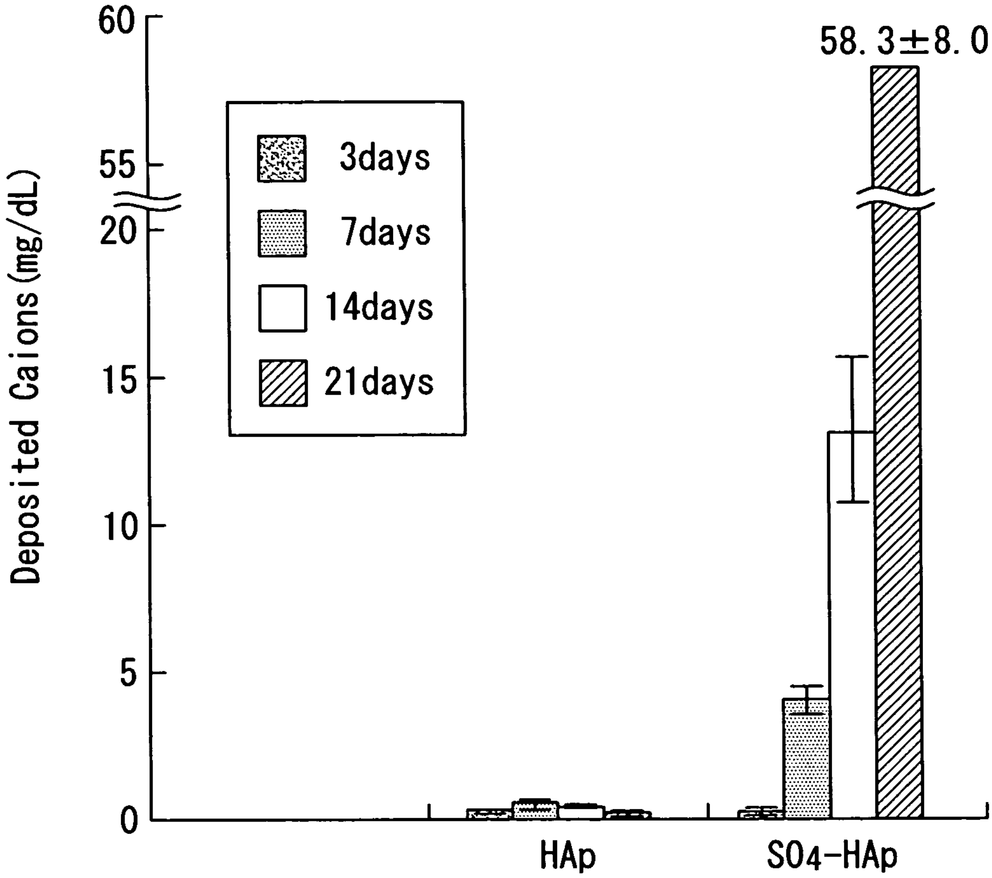
58.3±8.0
Deposited Caions (mg/dL)
60
55
20
15
10
5
0
3days
7days
14days
21days
HAp
SO4-HAp

FIG. 14

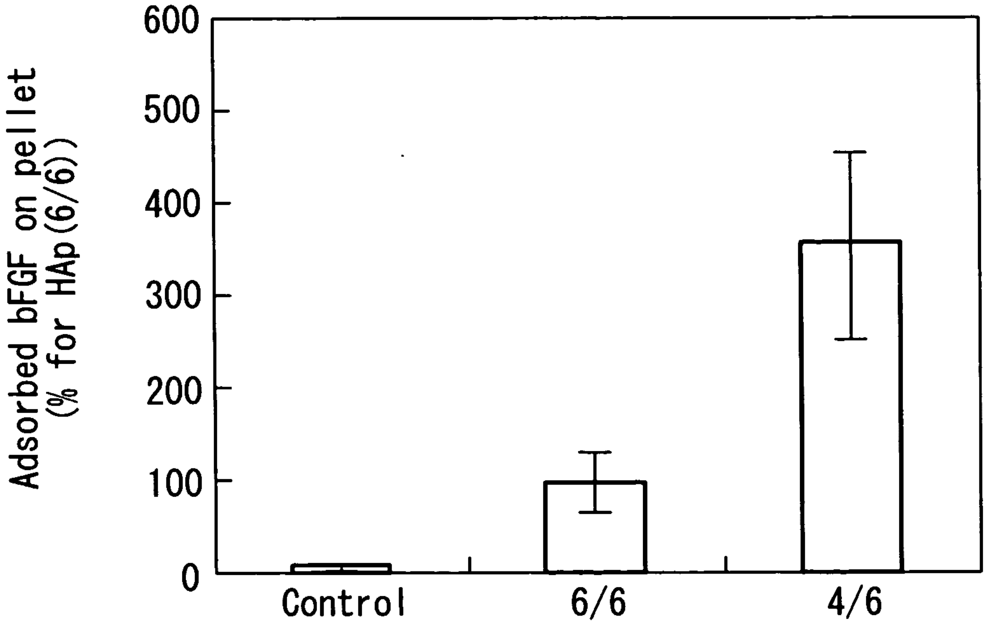
Adsorbed bFGF on pellet (% for HAp(6/6))
600
500
400
300
200
100
0
Control
6/6
4/6

**INTERNATIONAL SEARCH REPORT**

International application No. PCT/JP2006/314096

A. CLASSIFICATION OF SUBJECT MATTER
*A61L27/00*(2006.01)i, *C12N5/06*(2006.01)i

According to International Patent Classification (IPC) or to both national classification and IPC

B. FIELDS SEARCHED

Minimum documentation searched (classification system followed by classification symbols)
A61L27/00, C12N5/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
MEDLINE/CAPLUS/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | LIANG, Chong et al., Synthesis of calcium phosphate/calcium sulfate powder, Materials Chemistry and Physics, 2004, 88(2-3), 285-289 | 1-16 |
| A | JP 2000-508911 A (OSIRIS THERAPEUTICS INC), 18 July, 2000 (18.07.00), Full text & WO 97/40137 A1 & EP 906415 A1 & US 2003/031695 A1 | 4-16 |
| A | JP 2002-282285 A (Olympus Optical Co., Ltd.), 02 October, 2002 (02.10.02), Full text & WO 2002/76522 A1 & EP 1378256 A1 & US 2004/057939 A1 | 4-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 September, 2006 (22.09.06) | 03 October, 2006 (03.10.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/314096

C (Continuation). DOCUMENTS CONSIDERED TO BE RELEVANT

| Category* | Citation of document, with indication, where appropriate, of the relevant passages | Relevant to claim No. |
|---|---|---|
| A | LESHKIVICH, K. S. et al., Synthetic silicate sulfate apatite: Mechanical properties and biocompatibility testing, Journal of Materials Science: Materials in Medicine, 1993, 4(1), 86-94 (abstract) CAPLUS [online]; AMERICAN CHEMICAL SOCIETY (ACS) [retrieved on 1 September 2006] Retrieved from STN, CAPLUS Accession no.1993:656485 | 1-16 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **UEMURA.** Tissue engineering in bone using biodegradable βTCP porous material - A new material strengthened in vivo: Osferion. *Medical Asahi, The Asahi Shimbun Company,* 01 October 2001, vol. 30 (10), 46-49
- **RAFFY MIRZAYAN et al.** The use of calcium sulfate in the treatment of benign bone lesions. *The Journal of bone and joint surgery,* March 2001, vol. 83-A (3), 355-358